# FASCICULE DE BREVET EUROPEEN

(11) **EP 2 349 217 B1**
(45) Date de publication et mention de la délivrance du brevet: **31.07.2019**
(21) Numéro de dépôt: 09768176.1
(22) Date de dépôt: 10.11.2009
(51) Int. Cl.: A61K 9/16, A61K 31/191, A61K 47/34, A61K 47/36, A61P 25/32, A61K 9/50

(54) **Granulé d'acide gamma-hydroxybutyrique**
GAMMA-HYDROXYBUTTERSÄURE GRANULAT
Granule of gamma-hydroxybutyric acid

(30) Priorité: 14.11.2008 FR 0857763
(43) Date de publication de la demande: 03.08.2011
(73) Titulaire: Debregeas Et Associes Pharma, 75008 Paris (FR)
(72) Inventeur: LEBON, Christophe, F-28260 Rouvres (FR); SUPLIE, Pascal, F-27400 Montaure (FR)
(74) Mandataire: Gallochat, Alain
(86) Numéro de dépôt international: PCT/FR2009/052169
(87) Numéro de publication internationale: WO 2010/055260

(56) Documents cités:
- EP-A- 0 344 704
- EP-A- 0 635 265
- WO-A-93/00083
- WO-A2-00/69414
- FR-A1- 2 790 668
- US-A1- 2006 210 630
- US-A1- 2007 270 491
- US-B1- 6 436 438

## Description

La présente invention a pour objet une nouvelle composition à base d'acide gamma-hydroxybutyrique ou d'un de ses sels pharmaceutiquement acceptables, ainsi que le procédé de préparation de la composition susmentionnée.

L'acide γ-hydroxybutyrique (ou GHB) est considéré comme un neuromodulateur ou neurotransmetteur endogène présent dans le cerveau humain. C'est un métabolite de l'acide γ-aminobutyrique (GABA).

Ainsi, le médicament Xyrem® comprenant comme principe actif un sel de sodium du GHB, à savoir l'hydroxybutyrate de sodium (ou oxybate de sodium ; Na-GHB), est utilisé pour le traitement de la narcolepsie chez les patients adultes présentant une cataplexie.

Le GHB est également utilisé comme anesthésiant.

Il a également un effet mimétique de l'alcool sur le système nerveux central. Ainsi, des études cliniques ont déjà montré l'efficacité du GHB dans le traitement de la dépendance à l'alcool.

Le document EP 0 635 265 A décrit la préparation de compositions pharmaceutiques administrables par voie orale comprenant un coeur solide (sous la forme de granulé ou de comprimé) contenant l' acide gamma-hydroxybutyrique dispersé dans une matrice de type cellulosique (entre autre de l'hydropropylmethylcellulose) et un film de protection (entre autre un polymère méthacrylique de type eudragit) qui enrobe le coeur. Le document US 2006/210630 A1 décrit la préparation de pastilles comprenant un coeur contenant du NaGHB, de la cellulose microcristalline (Avicel) et du talc (un dérivé de silice) qui est enrobé par un agent entérique de type polymère méthacrylique (Eudragit). Le document US 2007/270491 A1 décrit des compositions pharmaceutiques aqueuses à base de gamma-hydroxybutyrate de sodium. L'utilisation de compositions à base de GHB dans le traitement de la catalepsie ou pour le sevrage de l' alcoolisme est mentionné au paragraphe [0003]. Le document EP 0 344 704 A décrit la préparation d'un sachet effervescent comprenant de l'acide gamma-hydroxybutyrique, du saccharose et des agents édulcorant/colorant (voir exemple 4) et d'un comprimé effervescent comprenant de l' acide gamma-hydroxybutyrique, du bicarbonate de sodium, de la cellulose micro cristalline, de la gesilite. Ces compositions orales solides sont utiles pour le sevrage de l'alcoolisme. Le document WO 93/00083 A décrit des compositions pharmaceutiques à base d'acide gamma-hydroxybutyrique.

Toutefois, l'inconvénient majeur du GHB en terme d'efficacité est lié à son profil pharmacocinétique. En effet, le GHB présente une demi-vie courte, un pic de concentration plasmatique élevé, une élimination rapide et une biodisponibilité variable (faible) en fonction de l'alimentation. Par exemple, il est connu que l'hydroxybutyrate de sodium est absorbé très rapidement par l'appareil gastroentérique avec un pic maximal d'environ 30-45 minutes après son administration et qu'il présente une demi-vie d'environ 20-25 minutes. De plus, son élimination est très rapide (en environ 4 à 5 heures).

Ce profil pharmacocinétique implique donc l'administration d'une dose journalière importante de 4 à 9 g, des prises répétées toutes les 3 à 4 heures, et en particulier au milieu de la nuit pour les patients narcoleptiques, ce qui a pour conséquence une efficacité limitée due aux grandes variations de concentration plasmatique ainsi qu'un risque d'intolérance dû à ces mêmes variations.

Les formes galéniques existantes ne permettent pas d'améliorer ce profil.

Par exemple, les solutions orales sont contraignantes en termes d'observance et peuvent générer des problèmes de stabilité et de conservation. De plus, étant donné que le GHB est instable en milieu acide, on ne peut exclure la possible dégradation en milieu gastrique du GHB et donc une diminution de la biodisponibilité.

La présente invention a pour but de fournir une nouvelle forme galénique à base d'acide gamma-hydroxybutyrique ou d'un de ses sels (notamment sodium) permettant de contourner les inconvénients susmentionnés.

Ainsi, la présente invention a pour but de fournir une nouvelle forme galénique à base d'acide gamma-hydroxybutyrique ou d'un de ses sels permettant de réduire la dose journalière et le nombre de prises quotidiennes, en augmentant la demi-vie apparente et la biodisponibilité du principe actif.

Ainsi, la présente invention a pour but de fournir une nouvelle forme galénique à base d'acide gamma-hydroxybutyrique ou d'un de ses sels permettant de diminuer ou supprimer les effets secondaires en réduisant les concentrations plasmatiques utilisées.

Ainsi, la présente invention a pour but de fournir une nouvelle forme galénique à base d'acide gamma-hydroxybutyrique ou d'un de ses sels permettant d'améliorer le confort du patient et le suivi du traitement en réduisant le nombre de prises journalières, en particulier en évitant les prises nocturnes.

Ainsi, la présente invention a pour but de fournir une nouvelle forme galénique à base d'acide gamma-hydroxybutyrique ou d'un de ses sels permettant d'améliorer la sécurité du produit par une forme galénique stable et évitant ou réduisant les détournements d'utilisation.

La présente invention concerne un granulé d'acide gamma-hydroxybutyrique ou d'un de ses sels pharmaceutiquement acceptables, caractérisé en ce qu'il comprend un coeur solide sur lequel est supporté l'acide gamma-hydroxybutyrique ou l'un de ses sels tel que défini dans les revendications 1-3.

L'invention est définie par l'objet des revendications 1-7.

De préférence, le principe actif utilisé est sous forme de sel, et plus particulièrement sous forme de sel de sodium (sel de sodium de l'acide 4-hydroxybutyrique ou oxybate de sodium).

L'expression "granulé" désigne une préparation constituée de grains solides secs, formant chacun un agrégat de particules de poudre d'une solidité suffisante pour permettre diverses manipulations.

Généralement, les granulés se présentent sous forme de petits grains de forme irrégulière et anguleuse. Or, les granulés selon la présente invention présentent la particularité de présenter une forme assez régulière, homogène et quasi sphérique.

Du point de vue physique, les granulés sont des agrégats de particules de poudres diverses cristallisées ou amorphes.

Les granulés de la présente invention sont destinés à une administration par voie orale, et plus particulièrement à être avalés tels quels.

Les granulés de la présente invention présentent une structure caractéristique de type coeur-écorce, le coeur n'étant pas de même nature que les principes actifs formant l'écorce.

Selon un mode de réalisation particulier, le coeur des granulés peut toutefois comprendre des particules d'acide γ-hydroxybutyrique ou d'un de ses sels.

Ainsi, ces granulés présentent une structure multicouche. En effet, le principe actif (acide γ-hydroxybutyrique ou l'un de ses sels) est déposé sur le coeur et donc forme une couche (ou écorce) déposée autour de ce coeur (ou support).

Chacun des documents US 6 436 438 B1, FR 2 790 668 A1 et WO 00/69414 A2 décrit la préparation de granulés comprenant un coeur inactif autour duquel est déposé le principe actif.

Le coeur des granulés peut également être considéré comme étant un support sur lequel vont se fixer les particules du principe actif.

Le coeur est constitué de particules solides et le principe actif supporté par ledit coeur est également sous forme solide.

La présente invention est donc basée sur la mise au point d'une nouvelle forme orale multi-particulaire.

Ainsi, l'originalité de la forme présentée ici consiste en un granulé destiné à la voie orale, permettant l'administration d'acide γ-hydroxybutyrique ou d'un de ses sels à des doses suffisamment importantes pour ne nécessiter qu'une ou deux administrations par jour, le granulé de l'invention étant fortement concentré en principe actif.

Les granulés de la présente invention présentent l'avantage de permettre une diminution, pour le patient, du nombre de prises journalières.

Selon un mode de réalisation préféré, le coeur des granulés de l'invention est constitué de particules d'un composé choisi dans le groupe constitué des polyols tels que le mannitol, le sorbitol, le maltitol ou le xylitol, du lactose, du phosphate dicalcique, des carbonates tels que le carbonate de calcium, de potassium, de magnésium ou de sodium, des gluconates, des silicates, notamment aminosilicate de magnésium (Neusilin®), des cristaux de sucre, du saccharose, des dérivés de la silice et des dérivés de l'amidon.

Selon un mode de réalisation particulièrement préféré, le coeur des granulés de l'invention est constitué de mannitol. De préférence, le coeur des granulés n'est pas constitué de noyaux neutres.

De préférence, la présente invention concerne donc des granulés comprenant de l'acide γ-hydroxybutyrique (ou l'un de ses sels) déposé sur un coeur constitué de particules de mannitol.

Selon un mode de réalisation particulièrement avantageux, le coeur des granulés selon la présente invention ne comprend pas de composé cellulosique.

Les granulés susmentionnés comprennent également un liant.

Le rôle du liant est de lier entre elles les particules, c'est-à-dire de parfaire la cohésion du granulé. Ainsi, les liants permettent d'assurer une bonne cohésion du principe actif et du coeur dans les granulés.

Ainsi, les liants se trouvent, comme le principe actif, déposés autour du coeur des granulés.

Comme liants, on peut citer la plupart des excipients hydrophiles qui donnent des solutions visqueuses : gommes arabique et adragante, méthylcellulose et carboxyméthylcellulose, gélatine, amidons, maltodextrines, PEG 4000 et 6000 en solution alcoolique, polyvidone en solution aqueuse ou alcoolique, et aussi des solutions de saccharose, de glucose ou de sorbitol.

Les liants des granulés de l'invention sont de préférence choisis dans le groupe constitué de l'amidon, du saccharose, de la gomme arabique, de la polyvinylpyrrolidone (PVP ou polyvidone), de l'hydroxypropylméthylcellulose (HPMC), de la gomme laque, de l'hydroxypropylcellulose (HPC), de la cellulose, des polyols ou des alginates, des glycérides polyglycolysés (Gelucire®) ou des macrogolglycérides, notamment macrogolglycérides de stéaroyle, également dérivés acryliques, ainsi que des mélanges de ceux-ci.

Parmi les polyols, on peut citer notamment le mannitol, le sorbitol, le maltitol ou le xylitol.

Selon un mode de réalisation particulier, les liants utilisés dans les granulés de la présente invention ne sont pas des composés cellulosiques. Ils sont donc de préférence choisis dans le groupe constitué de la polyvinylpyrrolidone, de la gomme laque, des polyols ou des alginates, des glycérides polyglycolysés (Gelucire) ou des macrogolglycérides, notamment macrogolglycérides de stéaroyle, ainsi que des mélanges de ceux-ci.

On peut également utiliser un liant choisi dans les groupes cités ci-dessus pour des propriétés particulières ; par exemple il peut être utile d'utiliser comme liant des excipients pH-dépendants tels que EUDRAGIT® L100 ou de la gomme laque. On peut également choisir d'utiliser préférentiellement des glycérides polyglycolysés (Gelucire®) pour leur caractère hydrophobe.

Selon un mode de réalisation préféré, les granulés de l'invention sont enrobés.

Les granulés enrobés sont constitués de grains enrobés d'une ou de plusieurs couches de mélanges d'excipients divers.

Ainsi, les granulés enrobés préférés selon la présente invention comprennent le principe actif déposé sur un coeur constitué de particules de mannitol, ainsi qu'une couche supplémentaire constituée de l'agent d'enrobage.

Selon un mode de réalisation préféré, les granulés de l'invention présentent une structure multicouche et sont constitués d'un coeur, de préférence à base de mannitol, sur lequel sont déposés le principe actif (GHB) et le liant, eux-mêmes enrobés d'une ou plusieurs couches d'agent(s) d'enrobage.

Les granulés de l'invention sont de préférence enrobés par un agent d'enrobage choisi dans le groupe constitué de la gomme laque, de la polyvinylpyrrolidone, du polyéthylène glycol (PEG), des dérivés cellulosiques tels que HPMC ou HPC, du saccharose, de l'alginate, des glycérides d'acides gras et des polymères méthacryliques.

Selon un mode de réalisation particulièrement préféré, les granulés de l'invention sont enrobés par de la gomme laque.

Les granulés de l'invention peuvent également être enrobés par un film d'enrobage dans lequel sont ajoutés un ou plusieurs excipients tels que des lubrifiants, des colorants, des édulcorants, des plastifiants ou des agents antiadhérant.

Les granulés de l'invention peuvent également comprendre un enrobage entérique pour une protection gastrique. De tels granulés sont donc gastrorésistants.

Un tel enrobage est obtenu avec des agents d'enrobage notamment constitué de HPMCP (hydroxypropylméthylcellulosephtalate - hypromellose phtalate) ou de polymères méthacryliques, notamment Eudragit® L, ou de gomme laque.

La présence de cet enrobage entérique peut influer sur la biodisponibilité du principe actif (GHB ou Na-GHB), notamment en évitant sa dégradation en milieu acide.

Les granulés de l'invention peuvent également comprendre un enrobage pour libération prolongée.

De tels granulés permettent une libération modifiée ou retardée des principes actifs (granulés à libération modifiée).

Un tel enrobage est obtenu avec des agents d'enrobage notamment constitués de copolymères de méthacrylates et d'acrylates Eudragit® S100, de gomme laque, de dérivés de la cellulose, notamment d'éthylcellulose, et de dérivés acryliques.

La présence de cet enrobage pour libération modifiée influence notamment la demi-vie apparente du principe actif (notamment GHB ou Na-GHB).

Les granulés selon la présente invention peuvent également comprendre un lubrifiant et/ou un arôme et/ou un édulcorant et/ou un colorant.

Parmi les lubrifiants mis en oeuvre dans le cadre de la présente invention, on peut notamment citer le talc, le stéarate de magnésium, les dérivés de silice (notamment Aerosil®) ou les cires.

Parmi les arômes mis en oeuvre dans le cadre de la présente invention, on peut citer les arômes classiquement utilisés dans les additifs alimentaires.

Les édulcorants mis en oeuvre dans le cadre de la présente invention sont notamment ceux listés dans la directive 94/35/CE du 30 juin 1994 concernant les édulcorants destinés à être employés dans les denrées alimentaires (modifiée par la directive 2006/25/CE du 5 juillet 2006). Ainsi, on peut notamment citer l'aspartame E 951, le sorbitol E 420, le mannitol E 421, l'acésulfame-K E 950 ou la saccharine E 954.

Les colorants mis en oeuvre dans le cadre de la présente invention sont notamment ceux listés dans la directive 95/45/CE du 26 juillet 1995 concernant les colorants pouvant être employés dans les denrées alimentaires (modifiée par la directive 2006/33/CE du 20 mars 2006). Ainsi, on peut notamment citer les colorants E 100 à E 180.

Pour éviter une prise involontaire du GHB dans le cadre d'utilisations détournées, il est particulièrement intéressant d'incorporer dans les granulés de l'invention un colorant qui se libère si ledit granulé est dissous ou broyé.

On peut également utiliser dans la formulation du granulé des excipients insolubles afin de prévenir la solubilisation totale du granulé pour usage détourné et malveillant.

Les granulés de l'invention peuvent également contenir un ou plusieurs plastifiants tels que ceux classiquement utilisés par l'homme du métier.

De préférence, un granulé selon la présente invention comprend au moins 35% en poids d'acide gamma-hydroxybutyrique ou d'un de ses sels, et préférentiellement au moins 45% en poids d'acide gamma-hydroxybutyrique ou d'un de ses sels.

Selon un autre mode de réalisation préféré, les granulés de la présente invention comprennent de 35% à 65% en poids d'acide gamma-hydroxybutyrique ou d'un de ses sels.

Par ailleurs, de préférence, le coeur d'un granulé de l'invention représente de 20% à 80%, et de préférence de 30% à 55%, voire de 35% à 55% en poids par rapport au poids total dudit granulé.

Les granulés de l'invention comprennent de préférence de 0 à 10%, et de préférence de 4 à 8%, en poids de liant.

Les granulés de l'invention comprennent de préférence de 10% à 45%, et de préférence de 20% à 30%, en poids d'agent d'enrobage.

Les granulés de l'invention comprennent de préférence moins de 3% en poids d'arôme.

Les granulés de l'invention comprennent de préférence moins de 2% en poids de colorant.

Les granulés de l'invention comprennent de préférence moins de 1,5% en poids d'édulcorant.

Les granulés de l'invention comprennent de préférence moins de 4% en poids de lubrifiant.

Les granulés de l'invention peuvent également comprendre un plastifiant, notamment du triéthylcitrate, à raison de moins de 3% en poids.

La présente invention concerne également une composition pharmaceutique, comprenant des granulés tels que définis ci-dessus.

La présente invention concerne également les granulés de l'invention tels que définis ci-dessus, pour leur utilisation pour le traitement de la catalepsie chez les patients narcoleptiques.

La présente invention concerne également les granulés de l'invention tels que définis ci-dessus, pour leur utilisation pour le sevrage de l'alcoolisme.

Les granulés selon l'invention peuvent être conditionnés dans des récipients individuels, par exemple dans des sachets, des sticks, des pochettes en papier ou des flacons, et de préférence dans des ampoules plastiques.

Les granulés de la présente invention présentent l'avantage de réduire le nombre de prises journalières. Ainsi, étant donné que les granulés de l'invention sont fortement dosés, la quantité d'acide γ-hydroxybutyrique (ou l'un de ses sels) par unité de prise (c'est-à-dire par récipient individuel contenant les granulés, notamment ampoule plastique) est de préférence supérieure ou égale à 500 mg, avantageusement supérieure ou égale à 1 g, et préférentiellement supérieure ou égale à 1,5 g.

Les granulés selon la présente invention peuvent être ingérés directement ou bien être dispersés dans une solution, ou mélangés dans un support alimentaire comme un yaourt ou une compote.

La présente invention concerne également un procédé de préparation d'un granulé tel que défini ci-dessus, caractérisé en ce qu'il comprend une étape d'application par poudrage de l'acide gamma-hydroxybutyrique ou d'un de ses sels sur un support particulaire solide.

Le procédé de l'invention consiste donc à mélanger le principe actif acide γ-hydroxybutyrique (ou l'un de ses sels) sous forme de poudre en présence de particules solides en tant que support. Ainsi, les particules solides du support utilisé forment un coeur sur lequel se déposent les particules du principe actif.

La structure des granulés de l'invention est donc liée à la mise en oeuvre de ce procédé particulier qui permet l'obtention de granulés de structure coeur-écorce.

En effectuant des essais comparatifs de préparation de granulés par un procédé de granulation directe avec différents excipients habituellement utilisés en granulation, il a été constaté que les résultats obtenus concernant le granulé lui-même sont satisfaisants concernant l'aspect, la friabilité et la dissolution. Cependant, les granulés obtenus par un tel procédé présentent une surface spécifique très élevée nécessitant des quantités importantes de polymères d'enrobage selon les techniques conventionnellement utilisées.

Ainsi, les granulés de la présente invention sont caractérisés en ce qu'ils présentent une surface spécifique abaissée. Par ailleurs, d'aspect, ils sont relativement lisses et présentent une forme plutôt régulière.

L'étape de poudrage susmentionnée du procédé pour la préparation des granulés de l'invention peut également comprendre une étape de pulvérisation d'une solution alcoolique ou hydroalcoolique ou aqueuse d'un liant.

Cette étape de pulvérisation et l'étape de poudrage sont de préférence effectuées de façon simultanée ou alternée.

De préférence, l'étape de poudrage susmentionnée est effectuée de façon concomitante avec une étape de pulvérisation d'un liant sous forme de solution.

La combinaison de ces étapes permet d'assurer une bonne cohésion du principe actif sur le coeur des granulés.

Une mise en oeuvre avantageuse du procédé de l'invention consiste ainsi à appliquer le principe actif sous forme de poudre sur le support particulaire susmentionné (ou coeur des granulés) en alternant des séquences de pulvérisation du liant sous forme de solution.

Le procédé de l'invention peut également comprendre, après l'étape de poudrage, une ou plusieurs étapes d'enrobage du granulé, notamment en déposant par pelliculage le ou les agents enrobants sous forme de films sur le granulé.

La faible surface spécifique des granulés de l'invention permet ainsi, en cas d'enrobage, de réduire la quantité utilisée d'agent d'enrobage et donc de moins diluer le principe actif dans lesdits granulés enrobés.

Un mode de réalisation préféré du procédé de l'invention consiste en un procédé comprenant, après l'étape d'enrobage, une étape de mélange avec un lubrifiant et/ou un arôme et/ou un édulcorant et/ou un colorant, ceux-ci pouvant être eux-mêmes préparés sous forme de granulés afin d'être finalement mélangés aux granulés actifs.

Toutefois les lubrifiants, arômes, édulcorants et colorants peuvent être également ajoutés avant l'étape de poudrage susmentionnée.

Si nécessaire, le procédé susmentionné peut également comprendre, avant l'étape de poudrage, une étape de broyage du principe actif (GHB) en présence d'un diluant.

Lorsque le procédé comprend une étape de broyage, celle-ci peut être suivie d'une étape d'addition des lubrifiants, arômes, édulcorants et colorants.

Ainsi, selon un mode de réalisation préféré, le procédé de préparation des granulés de l'invention comprend les étapes suivantes :
- une étape d'application par poudrage d'acide γ-hydroxybutyrique (ou d'un de ses sels pharmaceutiquement acceptables), mélangé avec au moins un diluant, sur un support particulaire solide, combinée à une étape de pulvérisation d'une solution alcoolique ou hydroalcoolique d'un liant, pour obtenir un granulé, ledit granulé étant constitué d'un coeur correspondant au support susmentionné sur lequel sont déposées des particules du principe actif ;
- une ou plusieurs étapes d'enrobage du granulé obtenu à l'étape précédente, par dépôt par pelliculage des films enrobants, pour obtenir un granulé enrobé ; et
- une étape facultative de mélange avec un lubrifiant et/ou un arôme et/ou un édulcorant et/ou un colorant, sous forme de granulé ou non.

### EXEMPLES

### Exemple 1 - Description détaillée d'un mode de réalisation préféré de préparation de granulés

Les ingrédients sont pesés un par un, ensuite le principe actif est introduit dans un mélangeur cubique. La quantité de diluant est à son tour pesée (mannitol 160) et introduite dans le mélangeur. Le mélangeur est ensuite mis en marche. Le mélange obtenu (A) est satisfaisant après 10 minutes.

Le mélange est introduit ensuite dans un broyeur Forplex FLO et l'intégralité du mélange est broyée de façon à réduire la granulométrie de l'ensemble (principe actif + diluant). Ceci permet d'augmenter la différence de taille des particules de mannitol (support)(environ 300 µ) et du mélange broyé (inférieur à 100 µ et préférentiellement de 25 µ).

L'étape suivante du procédé est une étape de poudrage mettant en oeuvre comme matériel une turbine conventionnelle.

Ainsi, le mannitol servant de support est introduit dans une cuve, celle-ci est alors mise en rotation (environ 20 tours par minute) et le mélange A est déposé par poudrage séquentiel sur le mannitol support, alternativement avec des phases de pulvérisation de la solution liante (PVP/HPMC/OH/H₂O).

Cette étape est faite de façon séquentielle afin de permettre l'évaporation et le séchage des granulés.

A l'issue de l'étape de poudrage, une phase de séchage est effectuée afin de faire circuler sur la masse de granulés de l'air chaud à environ 40°C pendant environ 14 heures.

A l'issue de l'étape de séchage, le produit est tamisé de façon à sélectionner les particules obtenues. Le mélange est ensuite replacé dans la cuve.

L'étape suivante est l'étape d'enrobage. Les solutions (ou suspensions) contenant les agents d'enrobage sont placées successivement dans une cuve basse pression sous agitation. La masse de granulés obtenue est alors placée dans la cuve d'un lit d'air fluidisé et les solutions d'enrobage sont alors pulvérisées successivement de façon continue sur les granulés. Des étapes de séchage/enrobage peuvent également être réalisées.

On utilise préférentiellement pour l'étape d'enrobage un appareil type lit d'air fluidisé (ou technologie apparentée) pour leur grande efficacité en terme d'évaporation, ce qui permet de réduire considérablement les temps d'enrobage.

On peut être également amené à réaliser différents types d'enrobage ayant chacun un rôle particulier, à savoir : consolidation, réalisation d'une couche hydrophobe, coloration, amérisation, modification de la libération du principe actif...

Par la suite, on peut ajouter aux granulés, dans un mélangeur, les additifs tels que les édulcorants, les lubrifiants, les arômes et les colorants sous forme de granulés ou non.

La dernière étape consiste à répartir les granulés dans les sachets individuels de conditionnement tels que des ampoules en plastique ou des sachets.

Les tableaux ci-après décrivent des exemples de granulés obtenus dans le cadre de la présente invention.

### Exemples 2 à 6 - Préparation de granulés selon l'invention

| **FORMULE N° 1** | | |
|---|---|---|
| Désignation | Fonction | Formules |
| | | Centésimale (%) |
| GHB | Principe Actif | 48,6 |
| Eudragit® L100 | Liant | 3,12 |
| saccharose | Coeur (support) | 20,77 |
| Aspartam | Edulcorant | 0,15 |
| Gomme laque | Enrobage | 19,23 |
| Neusilin® (Seppic) | Diluant | 4,22 |
| Talc | Lubrifiant | 3,85 |
| Colorant | Colorant | 0,06 |
| TOTAL | | 100,00 |

| **FORMULE N° 2** | | |
|---|---|---|
| Désignation | Fonction | Formules |
| | | Centésimale (%) |
| GHB | Principe Actif | 34,57 |
| PVP | Liant | 5,00 |
| Gélucire (Gattefossé) | Diluant | 10,00 |
| Neusilin® (Seppic) | Support | 21,79 |
| Hypromellose | Pré-enrobage | 2,00 |
| Eudragit® L30D | Enrobage | 21,89 |
| Triéthylcitrate | Plastifiant | 2,19 |
| Talc | Lubrifiant | 2,50 |
| Colorant | Colorant | 0,06 |
| TOTAL | | 100,00 |

| **FORMULE N° 3** | | |
|---|---|---|
| Désignation | Fonction | Formules |
| | | Centésimale (%) |
| GHB | Principe Actif | 45,09 |
| PVP | Liant | 4,51 |
| Carbonate de calcium | Support/tampon | 10,48 |
| Mannitol | Support (coeur) | 11,27 |
| Hypromellose | Pré-enrobage | 2,00 |
| Hypromellose Phtalate | Enrobage | 21,89 |
| Triéthylcitrate | Plastifiant | 2,19 |
| Talc | Lubrifiant | 2,50 |
| Colorant | Colorant | 0,06 |
| TOTAL | | 100,00 |

| **FORMULE N° 4** | | |
|---|---|---|
| Désignation | Fonction | Formules |
| | | Centésimale (%) |
| GHB | Principe Actif | 48,6 |
| EUD L100 | Liant | 2,9 |
| saccharose | Support (coeur) | 20,83 |
| Neusilin® (Seppic) | diluant | 4,37 |
| Eudragit® L100 | Enrobage | 18,35 |
| Triéthylcitrate | Plastifiant | 1,9 |
| Talc | Lubrifiant | 2,3 |
| Colorant | Colorant | 0,75 |
| TOTAL | | 100,00 |

| **FORMULE N° 5** | | |
|---|---|---|
| Désignation | Fonction | Formules |
| | | Centésimale (%) |
| GHB | Principe Actif | 46,33 |
| GLDB (gomme laque) | Liant | 4,55 |
| Mannitol | Coeur (support) | 19,85 |
| Neusilin® (Seppic) | Diluant | 4,17 |
| HP55® (phtalate d'hydroxypropyl méthyl cellulose) | Enrobage | 19,19 |
| Triéthylcitrate | Plastifiant | 1,92 |
| Aspartam | Edulcorant | 1,2 |
| Talc | Lubrifiant | 1,34 |
| Sepisperse vert | Colorant | 0,5 |
| TOTAL | | 100,00 |

## Revendications

1. Granulé d'acide gamma-hydroxybutyrique ou d'un de ses sels pharmaceutiquement acceptables, **caractérisé en ce qu'**il est constitué d'un coeur solide et d'une écorce déposée autour dudit coeur, ledit acide gamma-hydroxybutyrique ou l'un de ses sels pharmaceutiquement acceptables étant compris dans ladite écorce en présence d'un liant, et **en ce que** :
- ledit coeur est choisi dans le groupe constitué des polyols tels que le mannitol, le sorbitol, le maltitol ou le xylitol, du lactose, du phosphate dicalcique, des carbonates tels que le carbonate de calcium, de potassium, de magnésium ou de sodium, des gluconates, des silicates, des cristaux de sucre, du saccharose, de l'amidon, de la silice colloïdale, et de l'aluminosilicate de magnésium ;
- ledit liant est choisi dans le groupe constitué de l'amidon, du saccharose, de la gomme arabique, de la polyvinylpyrrolidone, de l'hydroxypropylmethylcellulose, de la gomme laque, de l'hydroxypropylcellulose, de la cellulose, des polyols, des alginates, de l'aluminosilicate de magnésium, des glycérides polyglycolyses ou des macrogolglycérides, notamment macrogolglycérides de stearoyle ;
- l'acide gamma-hydroxybutyrique ou l'un de ses sels pharmaceutiquement acceptables représentant au moins 35% en poids par rapport au poids total dudit granulé.

2. Granulé selon la revendication 1 **caractérisé en ce qu'**il est enrobé par un agent d'enrobage choisi dans le groupe constitué de la gomme laque, de la polyvinylpyrrolidone, du polyethylene glycol, de l'hydroxypropylméthylcellulose, de l'hydroxypropylcellulose, de l'éthylcellulose, du saccharose, de l'alginate, des glycérides d'acides gras et des polymères méthacryliques.

3. Granulé selon la revendication 2 **caractérisé en ce qu'**il comprend en outre un lubrifiant et/ou un arome et/ou un édulcorant et/ou un colorant.

4. Composition pharmaceutique, comprenant des granulés selon l'une quelconque des revendications 1 à 3.

5. Granulé selon l'une quelconque des revendications 1 à 3, pour son utilisation pour le traitement de la catalepsie chez les patients narcoleptiques, ou pour son utilisation pour le sevrage de l'alcoolisme.

6. Procédé de préparation d'un granulé selon l'une quelconque des revendications 1 à 3, **caractérisé en ce qu'**il comprend une étape d'application par poudrage de l'acide gamma-hydroxybutyrique ou d'un de ses sels sur un support particulaire solide, ladite étape de poudrage comprenant la pulvérisation d'une solution aqueuse, alcoolique ou hydroalcoolique d'un liant.

7. Procédé selon la revendication 6, **caractérisé en ce qu'**il comprend après l'étape de poudrage, une étape d'enrobage du granulé, notamment en déposant par pelliculage l'agent enrobant sous forme de film sur le granule, suivie, le cas échéant d'une étape de mélange avec un lubrifiant et/ou un arome et/ou un édulcorant et/ou un colorant.

## Patentansprüche

1. Pellet aus Gamma-Hydroxybuttersäure oder einem ihrer pharmazeutisch unbedenklichen Salze, **dadurch gekennzeichnet, dass** es aus einem festen Kern und einer um den Kern herum angeordneten Schalte besteht, wobei die Gamma-Hydroxybuttersäure oder eines ihrer pharmazeutisch unbedenklichen Salze in der Schale in Gegenwart eines Bindemittels enthalten ist, und dass
- der Kern aus der nachfolgenden Gruppe gewählt ist: Polyole wie z.B. Mannit, Sorbit, Maltit oder Xylit, Laktose, Dikalziumphosphat, Carbonate wie z.B. Kalzium-, Kalium-, Magnesium- oder Natriumcarbonat, Gluconate, Silikate, Zuckerkristalle, Saccharose, Stärke, kolloidales Siliziumdioxid und Magnesiumalumosilikat;
- das Bindemittel aus der nachfolgenden Gruppe gewählt ist: Stärke, Saccharose, Gummi arabicum, Polyvinylpyrrolidon, Hydroxypropylmethylcellulose, Schellack, Hydroxypropylcellulose, Cellulose, Polyole, Alginate, Magnesiumalumosilikat, polyglykolisierte Glyzeride oder Makrogolglyzeride, insbesondere Stearoyl-Makrogolglyzeride;
- wobei die Gamma-Hydroxybuttersäure oder eines ihrer pharmazeutisch unbedenklichen Salze bezogen auf das Gesamtgewicht des Pellets mindestens 35 Gew. % darstellt.

2. Pellet nach Anspruch 1, **dadurch gekennzeichnet, dass** es umhüllt ist mit einem Umhüllungsmittel, das aus der nachfolgenden Gruppe gewählt ist: Schellack, Polyvinylpyrrolidon, Polyethylenglykol, Hydroxypropylmethylcellulose, Hydroxypropylcellulose, Ethylcellulose, Saccharose, Alginat, Fettsäureglyzeride und Methacrylpolymere.

3. Pellet nach Anspruch 2, **dadurch gekennzeichnet, dass** es ferner ein Gleitmittel und/oder einen Aromastoff und/oder einen Süßstoff und/oder einen Farbstoff umfasst.

4. Pharmazeutische Zusammensetzung, umfassend Pellets nach einem der Ansprüche 1 - 3.

5. Pellet nach einem der Ansprüche 1 - 3 zur Verwendung bei der Behandlung der Katalepsie bei narkoleptischen Patienten oder zur Verwendung bei der Alkoholentwöhnung.

6. Verfahren zur Herstellung eines Pellets nach einem der Ansprüche 1 - 3, **dadurch gekennzeichnet, dass** das Verfahren umfasst: Auftragen von Gamma-Hydroxybuttersäure oder einem ihrer Salze auf einen feinteiligen Träger durch Abmehlen, wobei der Schritt des Abmehlens Sprühen einer wässrigen, alkoholischen oder hydroalkoholischen Lösung eines Bindemittels umfasst.

7. Verfahren nach Anspruch 6, **dadurch gekennzeichnet, dass** es nach dem Schritt des Aufmehlens umfasst: Umhüllen des Pellets, insbesondere durch Ablagern des Umhüllungsmittel in Filmform durch Beschichten auf das Pellet, ggf. gefolgt durch einen Schritt des Mischens mit einem Gleitmittel und/oder einem Aromastoff und/oder einem Süßstoff und/oder einem Farbstoff.

## Claims

1. Granule of gamma-hydroxybutyric acid or of a pharmaceutically-acceptable salt thereof, **characterized in that** it consists of a solid core and a shell deposited around the core, wherein the gamma-hydroxybutyric acid or one of its pharmaceutically-acceptable salts is comprised in the shell in the presence of a binder, and wherein:
- the core is selected from the group consisting of polyols such as mannitol, sorbitol, maltitol or xylitol, lactose, dicalcium phosphate, carbonates such as calcium carbonate, potassium carbonate, magnesium carbonate or sodium carbonate; gluconates, silicates, sugar crystals, sucrose, starch, colloidal silica, and magnesium aluminosilicate;
- the binder is chosen from the group consisting of starch, sucrose, gum arabic, polyvinylpyrrolidone, hydroxypropylmethylcellulose, shellac, hydroxypropylcellulose, cellulose, polyols, alginates, magnesium aluminosilicate, polyglycolysed glycerides or macrogolglycerides, especially stearoyl macrogolglycerides;
- gamma-hydroxybutyric acid or a pharmaceutically-acceptable salt thereof representing at least 35% by weight relative to the total weight of the granule.

2. Granule according to claim 1 **characterized in that** it is coated with a coating agent selected from the group consisting of shellac, polyvinylpyrrolidone, polyethylene glycol, hydroxypropylmethylcellulose, hydroxypropylcellulose, ethylcellulose, sucrose, alginate, glycerides of fatty acids and methacrylic polymers.

3. Granule according to claim 2 **characterized in that** it further comprises a lubricant and/or an aroma and/or a sweetener and/or a dye.

4. Pharmaceutical composition comprising granules according to any one of claims 1 to 3.

5. Granule according to any one of claims 1 to 3 for its use for the treatment of catalepsy in narcoleptic patients, or for its use for withdrawal from alcoholism.

6. Method for the preparation of a granule according to any one of claims 1 to 3, **characterized in that** it comprises an application step of powdering with gamma-hydroxybutyric acid or a salt thereof on a solid particulate support, wherein the powdering step comprises spraying an aqueous, alcoholic or aqueous-alcoholic solution of a binder.

7. Method according to claim 6, **characterized in that** it comprises after the powdering step, a step of coating the granule, in particular by depositing a coating of the coating agent in the form of a film on the granule, followed by a mixing step with a lubricant and/or an aroma and/or a sweetener and/or a dye, if appropriate.
